# EUROPEAN PATENT SPECIFICATION

(11) **EP 4 709 280 B1**
(45) Date of publication and mention of the grant of the patent: **19.08.2026**
(21) Application number: 24723485.9
(22) Date of filing: 28.04.2024
(51) Int. Cl.: A61B 6/00, G01R 33/20, G01R 33/54, A61B 6/58, G16H 40/40, G06Q 10/20, G05B 23/02, A61B 8/00, G16H 40/60

(54) **HEALTH STATUS OF IMAGING SYSTEM**
GESUNDHEITSSTATUS EINES BILDGEBUNGSSYSTEMS
ÉTAT DE SANTÉ D'UN SYSTÈME D'IMAGERIE

(30) Priority: 11.05.2023 EP 23172839
(43) Date of publication of application: 18.03.2026
(73) Proprietor: Koninklijke Philips N.V., 5656 AE Eindhoven (NL)
(72) Inventor: DIRKSEN, Peter, 5656 AG Eindhoven (NL); BARINK, Marco, 5656 AG Eindhoven (NL)
(74) Representative: Philips Intellectual Property & Standards
(86) International application number: PCT/EP2024/061703
(87) International publication number: WO 2024/231142

(56) References cited:
- EP-A1- 3 995 080
- US-A1- 2017 188 443
- US-A1- 2019 304 600

## Description

### FIELD OF THE INVENTION

The invention relates to the field of predictive maintenance, in particular to the field of determining a health metric of an imaging system. The invention relates to a computer implemented method for determining a health metric of an imaging system, a diagnostic system for determining a health metric of an imaging system, an X-ray source comprising such a diagnostic system, an imaging system comprising such a diagnostic system, a computer program element, and a computer readable medium.

### BACKGROUND OF THE INVENTION

The article "Test sequencing for sequential system diagnosis with precedence constraints and imperfect tests" by Wenchao Wei et al., in Decision Support Systems, Volume 103, November 2017, pages 104-116, discloses diagnostic testing procedure in which components are tested one by one, in a pre-specified order.

EP3995080A1 relates to a monitoring device for maintenance prediction for a medical imaging apparatus, a medical imaging apparatus comprising a monitoring device and a method of maintenance prediction for a medical imaging apparatus. The method comprises measuring a first data set of at least one parameter of the medical imaging apparatus by at least one sensor, wherein the measuring of the first data set is performed over a predetermined first period of measurement time, analysing , by a data processing unit, the first data set thereby determining a plurality of operation modes, in which the medical imaging apparatus was during the measurement time, based on the measured parameter, and measuring at least a second data set of the at least one parameter by the at least one sensor, wherein the measuring of the second data set is performed over a predetermined second period of measurement time.

US2019/0304600A1 describes a system comprising a sensing information acquisition unit that comprises at least one processing device, a processing device electrically coupled to a plurality of sensors and the plurality of sensors configured to detect functioning of medical diagnostic machine components. In an example, the system further comprises at least one end-user device configured to receive digital sensing data from at least one processing device and the at least one end-user device also configured to receive quality control (QA) data in digital imaging and communications in medicine (DICOM) format from the medical diagnostic machine components, the one end-user device comprises a data analytics platform configured to collect the sensing data and QA-DICOM data, the data analytics platform configured to determine the health of the medical diagnostic machine components and predict time to replace the medical diagnostic machine components by the collected digital sensing data and QA-DICOM data using specific matrix variables.

US2017/01888443A1 relates to an X-ray tube predictive fault indicator sensing device. A learning unit in learning mode generates a cluster from a cluster analysis of data formed from frequency constituent data and state data, obtained from a sensor unit. An abnormality calculation unit computes, as abnormalities, the minimum values among distances to surfaces of the clusters of the data formed from the frequency constituent data and the state data, obtained when in predictive fault indicator sensing mode. A predictive fault indicator determination unit determines a predictive fault indicator of an X-ray tube by comparing the abnormalities with a predetermined threshold.

Predictive maintenance aims to e.g. determine the condition of a machine, predict service intervals and remaining lifetime. By determining conditions and predicting events at an early stage, it may be possible to improve system uptime, extend system lifetime etc. To guarantee patient safety and throughput, predictive maintenance may be of particular importance for imaging systems, such as magnetic resonance imaging systems, X-ray imaging systems etc. In case of X-ray imaging systems, predictive maintenance may be used to identify or predict failure of e.g. the X-ray tube. As an example, failure categories may be extracted from signals recorded by vibration and audio sensors. However, imaging systems are generally very loud and noisy environments, with multiple sources of noise such as ventilators, pumps, power supplies etc. Therefore, reliable analysis of sensor data to extract system and/or component health may prove very difficult in practice. Hence, there is a need to improve predictive maintenance of (components of) imaging systems.

### SUMMARY OF THE INVENTION

It is an object of the invention to provide improved determination the health of an imaging system.

The invention is defined by the independent claims. Advantageous embodiments are defined by the dependent claims.

According to a first aspect of the invention, there is provided a computer implemented method for determining a health metric of an imaging system. The method comprises receiving sensor data acquired during a diagnostic sequence comprising multiple diagnostic intervals, wherein during each of the diagnostic intervals, a respective set of subsystems of the imaging system is active and a respective subset of sensor data is acquired, wherein the sets of subsystems active during the respective diagnostic intervals are mutually different. The method further comprises analyzing the subsets of sensor data to determine the health metric, wherein determining the health metric comprises performing a mathematical operation between at least two of the subsets of sensor data acquired during mutually different diagnostic intervals.

During the diagnostic intervals of the diagnostic sequence, various system components are switched on (or off) with a particular order and duration, and sensor data acquired during the different intervals can thereby be combined and corrected. A different set of subsystems of the imaging system is active during each of the diagnostic intervals, and a respective subset of sensor data is acquired during each of the diagnostic intervals. The mathematical operation may be e.g. subtraction, addition, multiplication, or division. This enables that the various contributions from the parts of the system to the sensor signals may be disentangled and compensated for. In this way, a cleaner and more relevant sensor data set may be extracted as starting point for determining health metrics and system health. E.g. in the case where the method aims to determine a status of an X-ray tube in a computed tomography system, contributions to the sensor signal from pumps, cooling, the gantry, power supplies etc. may be disentangled from signals relating to e.g. the rotational system of the X-ray tube anode. In this way, the reliability and level of detail of the analysis may be significantly improved compared to a measurement in 'steady state' operation of the system. Earlier detection and/or detection of smaller changes in relevant parameters may be achieved thanks to the cleaner signal. It is worth noticing that signals that may constitute noise for a metric may provide additional predictive or diagnostic information about the system health when disentangled. E.g. signals that originate from a fan, power supply or pump may be analyzed separately and tracked for potential failure analysis.

The diagnostic sequence may be a normal start-up procedure of the imaging system or a tailored diagnostic sequence. Preferably, the tailored diagnostic sequence may have longer intervals than a regular start-up procedure to enable more (accurate) sensor data collection during each interval. For example, the intervals may be 2-5 times longer than during a regular start-up sequence. A tailored diagnostic sequence may use different system and/or component parameters and/or order compared to a regular start-up sequence. The method may be routinely carried out in connection to normal usage of the system, such as before/after imaging. Alternatively or additionally, the method is carried out during service moments of the system. The imaging system may be a medical imaging system such as a computed tomography system, a fluoroscopy system, a radiography system, a magnetic resonance imaging system, positron emission tomography system, a single photon emission computed tomography system etc. The imaging system may also be e.g. an X-ray luggage screening system.

According to an embodiment of the invention, a first subset and a second subset comprise frequency data, and the mathematical operation comprises subtracting frequency data of the first subset from frequency data of the second subset. Frequency - time data may be derived from the sensor data using a Short-time Fourier Transform. By removing specific frequency peaks from a frequency spectrum, e.g. to separate static and dynamic frequencies during a time interval, and/or by correcting e.g. the average frequency between the subsets, determination of health metrics from sensor signals representing parameters such as vibration, rotation, sound etc. can be improved.

According to an embodiment of the invention, the method further comprises determining and outputting a health status based on the health metric, and/or determining and outputting a failure prediction based on the health metric, and/or determining and outputting a recommendation based on the health metric. The output of such information may enable e.g. a service engineer to swiftly take action where needed and may improve confidence in system uptime when output parameters are positive. Furthermore, such output may be used to trigger automatic changes to system settings, planning, ordering of parts etc. The recommendation may be a service recommendation.

According to an embodiment of the invention, the method further comprises analyzing the sensor data to automatically identify the type and/or duration of at least one diagnostic interval. By identifying the type and/or duration of diagnostic intervals from the sensor data itself, the method to determine the health metric may be (at least partly) autonomous. This may enable methods for improved predictive maintenance of e.g. replaceable components without additional connections or modifications to the imaging system software or hardware. As a non-limiting example, a replaceable X-ray tube for a computed tomography system or a C-arm system may have its own sensor board that can determine the health status of the tube independently of the imaging system, while still disentangling the contributions from the system to the sensor signals.

According to an embodiment of the invention, the method further comprises receiving a system trigger signal indicative of the type and/or duration of at least one diagnostic interval. In this example, the method is not (completely) autonomous from the imaging system as a trigger signal from the imaging system is received as part of the method. An embodiment with such a system trigger signal may be advantageous since the method may be enabled with e.g. fewer sensors, simplified data analysis and/or decreased data size.

According to an embodiment of the invention, the sensor data comprises data from a measurement of at least one of a sound and/or vibration, a temperature, an X-ray tube anode motor current, an X-ray emission, and an X-ray tube movement. The inventors have found that one or multiple of the above signals may be of particular importance when determining metrics of the health status of an imaging system such as an X-ray imaging system. Sensing of "X-ray tube movement" means sensing movement of the entire X-ray tube, such as in a rotating gantry or in a moving C-arm.

According to an embodiment of the invention, the diagnostic intervals comprise at least one of a first silent interval, an anode ramp-up interval, a gantry or C-arm ramp-up interval, an X-ray is on interval, an X-ray is off interval, a gantry or C-arm ramp-down interval, an anode ramp-down interval, and a second silent interval. The inventors have found that one or multiple of the above intervals may be of particular importance when determining metrics of the health status of an imaging system such as an X-ray imaging system. It is noted that in the context of the present invention the term "silent interval" means an interval where at least one component of the system is turned on but a component of interest for health assessment is turned off. E.g. the power, fans and/or cooling of the imaging system may be turned on but the component to monitor such as e.g. an X-ray tube is turned off. In this way, the silent interval may be used to collect background noise signals that may be subtracted from signals acquired during other diagnostic intervals. The first silent interval may be in the beginning of the diagnostic sequence and the second silent interval at the end of the diagnostic sequence. By comparing sensor signals under the same conditions during the beginning and end of the diagnostic sequence, possible hysteresis effects may be identified. In the context of the above-mentioned example intervals, "ramp-up" and "ramp-down" of the anode refers to respectively increasing and decreasing the rotational speed of an X-ray tube anode. Similarly, "ramp-up" and "ramp-down" of a gantry or C-arm refers to respectively increasing and decreasing the rotation/movement speed of the gantry or C-arm of an imaging system. During ramp-down, e.g. during ramp-down of an anode rotation, the rotation or movement may e.g. be allowed to slow down without breaking or it may be slowed down with breaking, or a combination may be applied, such as first slow down without breaking and after a time period brake. In a diagnostic sequence, such as a tailored diagnostic sequence, a rotation or movement may be at a different speed than during normal usage. E.g. at a lower speed to shorten a slow-down period.

According to an embodiment of the invention, the health metric comprises at least one of a change in anode motor current, an average frequency, integer orders, non-integer orders, sound ticks, and a linewidth of measured X-ray. These health metrics may provide valuable insights to the health status of the system. E.g. changes in anode motor current may be an early indicator for end-of life of the anode rotational system. Increased average frequency provides early warning of failure. Integer orders may indicate e.g. looseness in a motor shaft. Non-integer orders may provide early warning of bearing failure. Sound ticks, particularly high frequency sound ticks, may indicate X-ray tube arcing. Linewidth of an X-ray sensor may indicate stability of rotational speed during X-ray operation, etc.

According to a second aspect, there is provided a diagnostic system for determining a health metric of an imaging system, the diagnostic system comprising a sensor for acquiring sensor data and a processor configured to carry out the method of the first aspect. The processor may be configured to determine a health status based on the health metric. The processor may comprise a short-term and/or long-term memory. The processor and the sensor may be located in physical proximity, such as on a sensor board. Alternatively or additionally, the diagnostic system may comprise a distributed system where processing is carried out at e.g. a remote server, a separate computing device etc. Data such as sensor data, health metrics, health status data etc. may be stored locally and/or remotely from the sensor. In an example pre-processing of data is done on a sensor board. In such an example, only pre-processed data may be stored, which may reduce data storage. As an example, raw sensor data may be discarded if parameters are within certain ranges, and only when a threshold is passed, more raw data is collected e.g. for a more accurate analysis.

According to an embodiment of the invention, the diagnostic system comprises a communication unit configured to communicate at least one of a health metric, a health status, a failure prediction, and a service recommendation. The communication unit may communicate the information to a service interface such as a local or remote user interface, computer, handheld device etc. The communication may trigger an alarm, such as a visible, haptic and/or audible alarm.

According to an embodiment of the invention, the sensor is configured to be attached to an X-ray source. When monitoring or determining a health metric of an X-ray source, such as an X-ray tube, it may be advantageous to attach a sensor directly to the X-ray source. This may be particularly useful when sensing sound or vibrations. The location of the sensor on the source, attachment means such as screws etc. may be optimized to improve signal to noise ratio of the sensor signal.

According to an embodiment of the invention, the sensor comprises one or more of a piezo sensor, a microelectromechanical systems (MEMS) sensor, an ultrasound sensor, a current sensor, an X-ray sensor and a gyro sensor.

According to a third aspect an X-ray source is provided, wherein the X-ray source comprises the diagnostic system. The X-ray source may be an X-ray tube, such as a rotating anode X-ray tube.

According to a fourth aspect an imaging system is provided, wherein the imaging system comprises the diagnostic system. The imaging system may be a medical imaging system such as a computed tomography system, a fluoroscopy system, a radiography system, a magnetic resonance imaging system, positron emission tomography system, a single photon emission computed tomography system etc. The imaging system may also be e.g. an X-ray luggage screening system.

According to a fifth aspect there is provided a computer program element, which, when being executed by a processor, is adapted to cause the processor to perform the method according to the first aspect.

According to a sixth aspect there is provided a computer readable medium having stored thereon the computer program element as mentioned above.

The computer program element might be stored on a computer unit, which might also be part of an embodiment. This computing unit may be configured to perform or induce performing of the steps of the method described above. Moreover, it may be configured to operate the components of the above-described imaging system. The computing unit can be configured to operate automatically and/or to execute the orders of a user. A computer program may be loaded into a working memory of a data processor. The data processor may thus be equipped to carry out the method according to one of the preceding embodiments. An exemplary embodiment of the invention covers both a computer program that right from the beginning uses the invention, and a computer program that by means of an update turns an existing program into a program that uses the invention. Further on, the computer program element might be able to provide all necessary steps to fulfill the procedure of an exemplary embodiment of the method as described above. A computer program may be stored and/or distributed on a suitable medium, such as an optical storage medium or a solid-state medium supplied together with or as part of other hardware, but may also be distributed in other forms, such as via the internet or other wired or wireless telecommunication systems. The computer program may be presented over a network like the World Wide Web and can be downloaded into the working memory of a data processor from such a network.

These and other aspects of the invention will be apparent from and elucidated with reference to the embodiments described hereinafter.

### BRIEF DESCRIPTION OF THE DRAWINGS

Fig. 1 shows a flow chart of a method for determining a health metric of an imaging system.
Fig. 2 shows a flow chart of a method for determining a health metric of an imaging system.
Fig. 3 shows a flow chart of a method for determining a health metric of an imaging system.
Fig. 4 schematically illustrates a diagnostic system for determining a health metric of an imaging system.
Fig. 5 illustrates an example of sensor signals during different diagnostic intervals.

### DESCRIPTION OF EMBODIMENTS

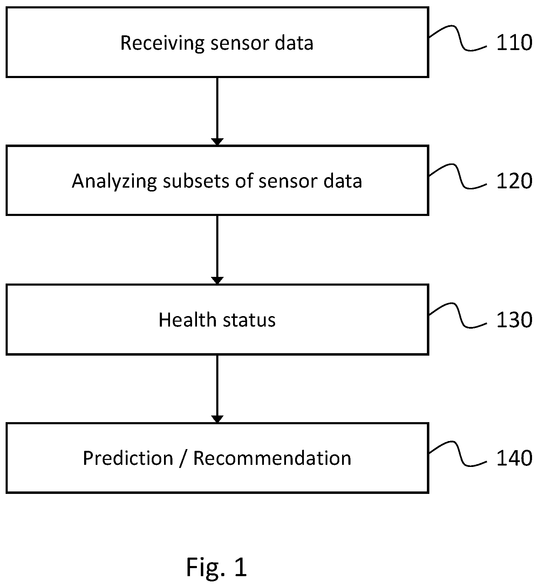

The flow chart in Fig. 1 illustrates an example of a computer implemented method for determining a health metric and optionally a health status of an imaging system. In a first step, the method includes receiving 110 sensor data. The sensor data is acquired during a diagnostic sequence comprising multiple diagnostic intervals, wherein a different set of subsystems of the imaging system are active during each of the diagnostic intervals, and wherein and a subset of sensor data is acquired during each of the diagnostic intervals. The sensor data may include data from measurements of parameters such as sound, vibration, and/or temperature. If the imaging system is an X-ray imaging system, the sensor data may include data from measurements of e.g. X-ray tube anode motor current, X-ray emission, and/or X-ray tube movement. The subsets of sensor data are analyzed 120 to determine one or more health metrics based on a combination of subsets of sensor data acquired during different diagnostic intervals by performing a mathematical operation between at least two subsets of sensor data acquired during mutually different diagnostic intervals. As an example, sensor data from an interval identified as a silent interval may be subtracted as background from sensor data acquired during a subsequent interval. In this way, the influence from other components of the system can be disentangled and corrected for, and the health metric can be determined from a 'clean' signal. Herewith, small changes and/or absolute values of health metrics may be detected with high reliability. Examples of health metrics may include metrics such as a change in anode motor current, an average frequency, integer orders, non-integer orders, sound ticks, and/or a linewidth of measured X-ray. In a following step, a health status of the imaging system may be determined 130 based on the one or more health metrics. As an example, a health status may indicate the health of the system and/or a component of the system on a scale. The scale may comprise a (numerical) range from 'good' to 'bad', and/or discrete indications of the status. Alternatively or additionally, the health status may comprise an indicator or a warning of (upcoming) component or system failure, stability, etc. In other words, the one or more health metrics determined from the analyzed sensor data in the previous step 120, may be used in the subsequent step 130 to indicate a current or future health status. Whereas the health metrics are linked to specific measurements by the sensors, the health status may be more actionable in the form of health indications, warnings, alerts etc. The health status may be output to e.g. a user interface. Alternatively or additionally, the method may also comprise a step 140 of determining and outputting a failure prediction based on the health metric, and/or determining and outputting a service recommendation based on the health metric. By providing predictions and/or recommendations, the output may be further actionable to a user, such as a service engineer. Predictions and/or recommendations may be used to trigger (semi)automatic actions such as adapted system or device settings, (suggested) ordering of components, adapted procedure planning or workflows etc.

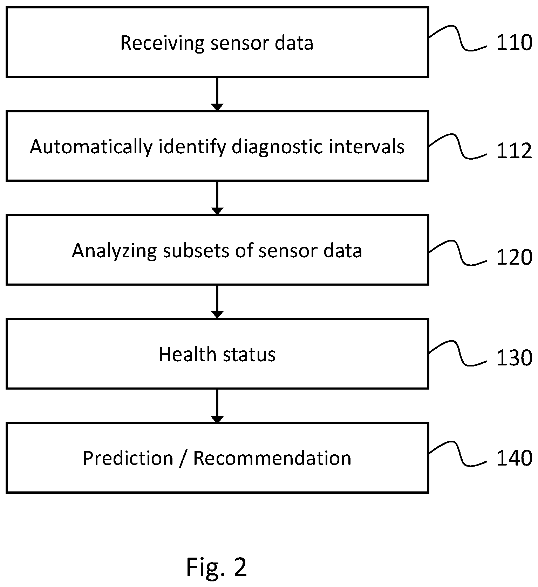

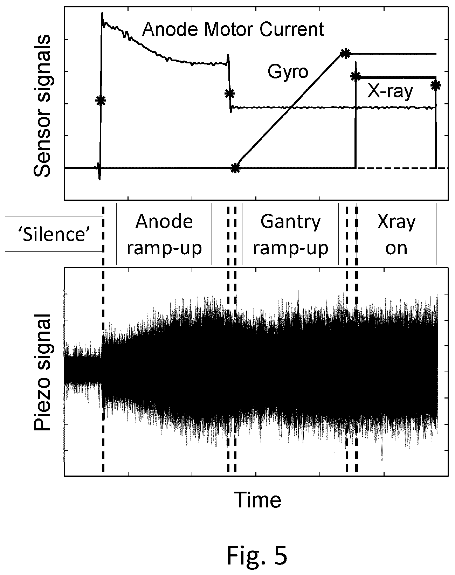

Fig. 2 shows an example of a method comparable to the method described above. In the example in Fig. 2, the method includes analyzing sensor data to automatically identify 112 the type and/or duration of one or more diagnostic intervals. This is further exemplified by the sensor signals illustrated in Fig. 5. By identifying the type and/or duration of diagnostic intervals from the sensor data itself, the method to determine the health status may be (at least partly) autonomous. This may enable methods for improved predictive maintenance of e.g. replaceable components without additional connections or modifications to the imaging system software or hardware.

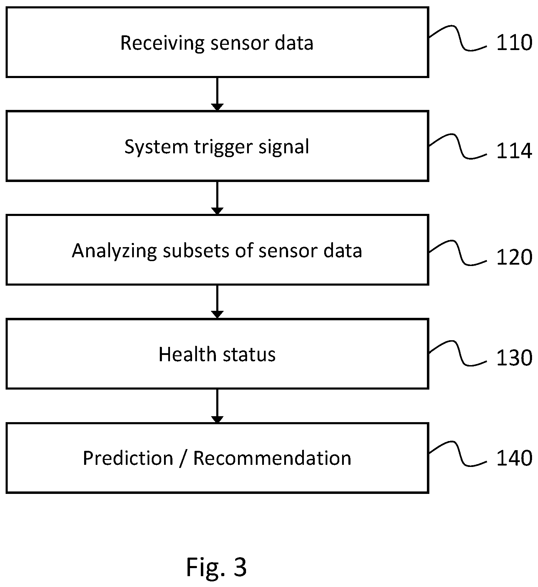

Fig. 3 shows an example of a method that includes receiving 114 a system trigger signal indicative of the type and/or duration of at least one diagnostic interval. In this example, the method is not (completely) autonomous from the imaging system as a trigger signal from the imaging system is received as part of the method. An embodiment with such a system trigger signal may be advantageous since the method may be enabled with e.g. fewer sensors, simplified data analysis and/or decreased data size.

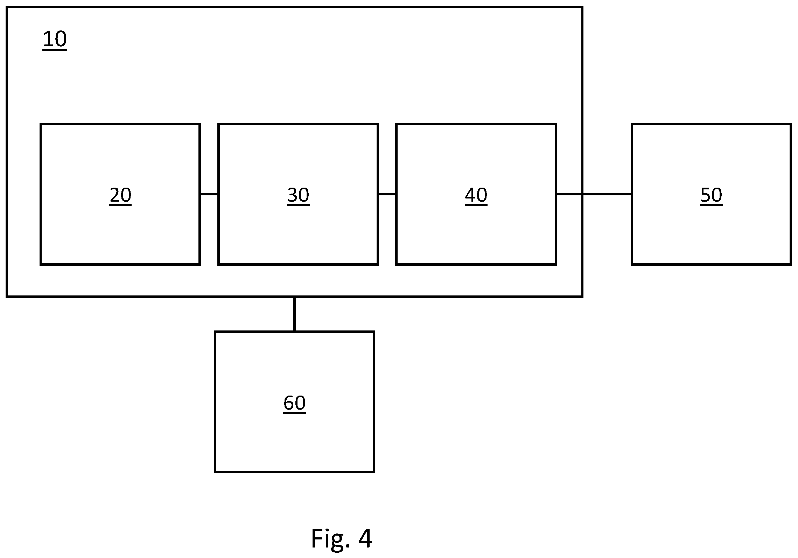

Fig. 4 schematically illustrates an example of a diagnostic system 10 for determining a health metric of an imaging system 60. The diagnostic system 10 may be comprised in the imaging system 60 or be a separate system. A diagnostic system 10 for determining a health metric of an X-ray imaging system may be comprised in an X-ray source for use in the X-ray imaging system. Connections between the systems and/or parts of the systems may be wired and/or wireless.

In the example in Fig. 4, the diagnostic system 10 includes a sensor 20, a processor 30 and a communication unit 40. The sensor 20 may be located in or in the proximity of the imaging system 60 or even attached to a component or a part of the imaging system 60. The diagnostic system 10 and the imaging system 60 may comprise one or several common sensors 20. The processor 30 may comprise a short-term and/or long-term memory. The processor 30 may be connected to the imaging system 60. The processor 30 may be configured to receive a signal from the imaging system 60, such as a signal from a CPU, encoder etc. of the imaging system 60. The sensor 20 is configured to acquire sensor data from the imaging system 60. The processor 30 is configured to carry out a method of determining a health metric of the imaging system, such as the methods exemplified in Figs. 1-3. The processor 30 may be configured to determine a health status, failure prediction, service recommendation etc.

In the example in Fig. 4, the communication unit 40 is connected to a service interface 50. The service interface may be a local or remote user interface, computer, handheld device etc. Examples of user interfaces include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable user interaction in a manner free from constraints imposed by input devices such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth. Via the service interface 50, the diagnostic system 10 may communicate e.g. a health metric, a health status, a health prediction, a recommendation, an alarm, a trigger for automatic system actions etc.

Fig. 5 illustrates sensor signals during analysis of a health status of a computed tomography system, which includes a rotating anode X-ray tube. The computed tomography system may be a regular computed tomography system but with the addition of a diagnostic system 10. The bottom part of the figure shows an example of a piezo vibration signal recorded during a 1-minute system start-up. The top part illustrates the signal from three additional sensors during the same sequence. The diagnostic system 10 may record signals from multiple sensors such as an anode motor current sensor, a gyro, a MEMS, a piezo, an ultrasound sensor, an X-ray detector, a temperature sensor etc. In the example in Fig. 5, the signal is measured from an anode motor current sensor, a gyro, an X-ray detector and a piezo sensor. From the sensor signals, change points, i.e., the moments when a subsystem switches on/off, and the respective diagnostic intervals are identified. In this case four intervals are identified: 1) 'silence' when the X-ray tube and gantry are switched off, 2) 'anode ramp-up', where an anode motor switches on and ramps up until it reaches its steady state RPM, 3) 'gantry ramp-up' where the rotation of the gantry (in which the X-ray tube is located) is ramped up and finally 4) a time interval with 'X-ray on', i.e. X-rays are generated with the X-ray tube. In this example, the diagnostic system 10 operates autonomously, and there are no system trigger signals available. As shown from the top part of the figure the change points and intervals may be identified using the motor current, gyro, and X-ray sensor. Knowing these intervals is important for the data analysis. Additionally, ramp-down intervals and/or a second silent interval (not shown) may also be used for the analysis. With information about the diagnostic intervals and the sensor subsets, several health metrics may be determined. These health metrics may be used to determine one or more health statuses, in this case relating to the X-ray tube, gantry etc.

An example of a health metric are the parameters that describe an envelope of the anode motor current during the anode ramp up time interval. The motor current curve during may be characterized by a time constant. As the tube ages and there is wear to the rotational system (including bearings) of the anode, the time constant changes. Therefore, this health metric may be used to determine a health status that indicates wear and/or predicted end of lifetime for the tube. By combining sensor data from the ramp-up interval with sensor data from a ramp-down interval, e.g. without breaking the anode, earlier detection of tube deterioration may be achieved. A second example of a health metric is the mean frequency. Changes in the mean frequency are related to health status of the tube, gantry or other parts of the system. As an example, high frequency components of the signal may increase, causing an overall increase of the mean frequency. Frequency peaks e.g. determined during the time interval 'silence' are used to correct for background noise during other diagnostic intervals. Short-time Fourier Transform may be used to create a frequency - time dataset for this purpose. A third health metric is an order spectrum. Order analysis may be a powerful tool to extract vibration components that have a fixed rate with respect to the anode motor RPM. Sharp peaks in the order spectrum represent components that have a fixed rate with respect to the changing RPM, so-called orders. Orders may be used to determine (upcoming) bearing failure, shaft looseness. Time interval 'silence' may be used to identify and remove frequency signals not related to the anode ramp-up and changing RPM. A fourth health metric is electrical arcing. E.g. a piezo sensor signal detects a characteristic ticking sound that arcs produce. This signal is measured during the time interval 'Xray-on'. A fifth metric is the line width of the Xray-sensor, which relates to RPM stability when Xray is on. This measure may be combined with other measurements of the X-ray tube RPM map.

In Fig. 5, the piezo signal and identified change points may be used to determine the RPM map of the rotating anode. An important first step is the subtraction of the background signal, as determined from the 'silence' time interval. E.g., frequency peaks that are not from the system components of interest and/or independent of the anode rotation may be corrected for. This may be of particular importance when anode rotation frequencies and resonance frequencies of other components are relatively similar.

The change points may be provided by the imaging system 60 as trigger signals instead. In such a case, it may be possible to do the analysis with fewer sensors 20, thereby simplifying the analysis and decreasing the data size. E.g. using only the piezo sensor (bottom part of the figure) and known change points it is possible to determine the RPM map, order spectrum and the mean frequency. The piezo sensor may operate during the all the time intervals, also when the gantry rotates at full speed of e.g. 2 Hz. In this way, the measurement of the mean frequency also captures changes of vibration frequencies from fans, cooling pump, power supply etc. during 'silence', the X-ray tube during anode ramp-up, as well as from the gantry and X-ray sub system in the last phase of a system start-up. Hence, by disentangling the piezo sensor signal between the diagnostic intervals, it is possible to determine multiple health statuses relating to different parts of the imaging system 60. As an example, the difference in mean steady state frequency at the end of anode ramp-up and mean frequency during gantry ramp-up may provide information about the system health of the gantry.

The sensors may e.g. be on a separate sensor board or a series of sensors integrated into the X-ray system. Additional sensors may also be used for the analysis, such as (X-ray) sensors that are already present in the imaging system. Both a start-up and a run-down (not shown in Fig. 5) may be included in the data set and analysis for a better comparison of sensor subsets. In such a case, it may be advantageous that start-up and run-down have the same conditions in order to improve the repeatability of the health metrics and health status determination. Possible hysteresis effects may also provide valuable insights to system health.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. Computer implemented method for determining a health metric of an imaging system (60), the method comprising:
receiving (110) sensor data acquired during a diagnostic sequence comprising multiple diagnostic intervals, wherein during each of the diagnostic intervals, a respective set of subsystems of the imaging system (60) is active and a respective subset of sensor data is acquired, wherein the sets of subsystems active during the respective diagnostic intervals are mutually different; and
analyzing (120) the subsets of sensor data to determine the health metric, wherein determining the health metric comprises performing a mathematical operation between at least two of the subsets of sensor data acquired during mutually different diagnostic intervals with mutually different sets of subsystems active.

2. The method according to claim 1, wherein a first subset and a second subset comprise frequency data, and wherein the mathematical operation comprises subtracting frequency data of the first subset from frequency data of the second subset.

3. The method according to claim 1 or 2, wherein the method further comprises determining and outputting (130) a health status based on the health metric, and/or determining and outputting (140) a failure prediction based on the health metric, and/or determining and outputting (140) a recommendation based on the health metric.

4. The method according to claim 1 or 2 or 3, wherein the method further comprises analyzing the sensor data to automatically identify (112) the type and/or duration of at least one diagnostic interval.

5. The method according to any of the preceding claims, wherein the method further comprises receiving (114) a system trigger signal indicative of the type and/or duration of at least one diagnostic interval.

6. The method according to any of the preceding claims, wherein the sensor data comprises data from a measurement of at least one of
a sound and/or vibration;
a temperature;
an X-ray tube anode motor current;
an X-ray emission; and
an X-ray tube movement.

7. The method according to any of the preceding claims, wherein the diagnostic intervals comprise at least one of
a first silent interval;
an anode ramp-up interval;
a gantry or C-arm ramp-up interval;
an X-ray is on interval;
an X-ray is off interval;
a gantry or C-arm ramp-down interval;
an anode ramp-down interval; and
a second silent interval

8. The method according to any of the preceding claims, wherein the health metric comprises at least one of
a change in anode motor current;
an average frequency;
integer orders;
non-integer orders;
sound ticks; and
a linewidth of measured X-ray.

9. Diagnostic system (10) for determining a health metric of an imaging system (60), the diagnostic system (10) comprising:
a sensor (20) for acquiring sensor data, and
a processor (30) configured to carry out the method according to any of the preceding claims.

10. The diagnostic system (10) according to claim 9, wherein the diagnostic system (10) comprises a communication unit (40) configured to communicate at least one of a health metric, a health status, failure prediction, and a service recommendation.

11. The diagnostic system (10) according to claim 9 or 10, wherein the sensor (20) is configured to be attached to an X-ray source.

12. The diagnostic system (10) according to claim 9 or 10 or 11, wherein the sensor (20) comprises one or more of a piezo sensor, a mems sensor, an ultrasound sensor, a current sensor, an X-ray sensor and a gyro sensor.

13. Imaging system (60) comprising the diagnostic system (10) according to any of claims 9 to 12.

14. A computer program element, which, when being executed by a processor (30), is adapted to cause the processor (30) to perform the method according to any of claims 1 to 8.

15. A computer readable medium having stored thereon the computer program element of claim 14.

## Patentansprüche

1. Computerimplementiertes Verfahren zur Bestimmung einer Zustandsmetrik eines Bildgebungssystems (60), das Verfahren umfassend:
Empfangen (110) von Sensordaten, die während einer Diagnosesequenz, die mehrere Diagnoseintervalle umfasst, erfasst wurden, wobei während jedes der Diagnoseintervalle ein jeweiliger Satz von Teilsystemen des Bildgebungssystems (60) aktiv ist und ein jeweiliger Teilsatz von Sensordaten erfasst wird, wobei sich die während der jeweiligen Diagnoseintervalle aktiven Sätze von Teilsystemen voneinander unterscheiden; und
Analysieren (120) der Teilsätze von Sensordaten, um die Zustandsmetrik zu bestimmen, wobei das Bestimmen der Zustandsmetrik Durchführen einer mathematischen Operation zwischen mindestens zwei der Teilsätze von Sensordaten umfasst, die während voneinander unterschiedlicher Diagnoseintervalle mit voneinander unterschiedlichen Sätzen von aktiven Teilsystemen erfasst wurden.

2. Verfahren nach Anspruch 1, wobei ein erster Teilsatz und ein zweiter Teilsatz Frequenzdaten umfassen und wobei die mathematische Operation Subtrahieren von Frequenzdaten des ersten Teilsatzes von Frequenzdaten des zweiten Teilsatzes umfasst.

3. Verfahren nach Anspruch 1 oder 2, wobei das Verfahren weiter Bestimmen und Ausgeben (130) eines Zustandsstatus auf der Grundlage der Zustandsmetrik und/oder Bestimmen und Ausgeben (140) einer Ausfallvorhersage auf der Grundlage der Zustandsmetrik und/oder Bestimmen und Ausgeben (140) einer Empfehlung auf der Grundlage der Zustandsmetrik umfasst.

4. Verfahren nach Anspruch 1 oder 2 oder 3, wobei das Verfahren weiter Analysieren der Sensordaten zum automatischen Identifizieren (112) des Typs und/oder der Dauer von mindestens einem Diagnoseintervall umfasst.

5. Verfahren nach einem der vorstehenden Ansprüche, wobei das Verfahren weiter Empfangen (114) eines Systemauslösesignals umfasst, das den Typ und/oder die Dauer von mindestens einem Diagnoseintervall angibt.

6. Verfahren nach einem der vorstehenden Ansprüche, wobei die Sensordaten Daten aus einer Messung umfassen, von mindestens einem von
einem Geräusch und/oder einer Vibration;
einer Temperatur;
einem Anodenmotorstrom einer Röntgenröhre;
einer Röntgenstrahlungsemission; und
einer Röntgenröhrenbewegung.

7. Verfahren nach einem der vorstehenden Ansprüche, wobei die Diagnoseintervalle mindestens eines umfassen von
einem ersten stillen Intervall;
einem Anoden-Anlaufintervall;
einem Anlaufintervall für die Signalbrücke oder den C-Bogen;
einer Röntgenstrahlung im Intervall;
einer Röntgenstrahlung außerhalb des Intervalls;
einem Herunterfahrintervall für die Signalbrücke oder den C-Bogen;
einem Anoden-Herunterfahrintervall; und
einem zweiten stillen Intervall.

8. Verfahren nach einem der vorstehenden Ansprüche, wobei die Zustandsmetrik mindestens eines umfasst von
einer Änderung des Anodenmotorstroms;
einer durchschnittlichen Frequenz;
ganzzahligen Ordnungen;
nicht-ganzzahligen Ordnungen;
Tonsignalen; und
einer Linienbreite der gemessenen Röntgenstrahlung.

9. Diagnosesystem (10) zur Bestimmung einer Zustandsmetrik eines Bildgebungssystems (60), das Diagnosesystem (10) umfassend:
einen Sensor (20) zur Erfassung von Sensordaten, und
einen Prozessor (30), der so ausgebildet ist, dass er das Verfahren nach einem der vorstehenden Ansprüche ausführt.

10. Diagnosesystem (10) nach Anspruch 9, wobei das Diagnosesystem (10) eine Kommunikationseinheit (40) umfasst, die so ausgebildet ist, dass sie mindestens eines kommuniziert von einer Zustandsmetrik, einem Zustandsstatus, einer Ausfallvorhersage und einer Wartungsempfehlung.

11. Diagnosesystem (10) nach Anspruch 9 oder 10, wobei der Sensor (20) ausgebildet ist, um an einer Röntgenquelle angebracht zu werden.

12. Diagnosesystem (10) nach Anspruch 9 oder 10 oder 11, wobei der Sensor (20) einen oder mehrere von einem Piezosensor, einem MEMS-Sensor, einem Ultraschallsensor, einem Stromsensor, einem Röntgensensor und einem Gyrosensor umfasst.

13. Bildgebungssystem (60), umfassend das Diagnosesystem (10) nach einem der Ansprüche 9 bis 12.

14. Computerprogrammelement, das, wenn es von mindestens einem Prozessor (30) ausgeführt wird, dazu angepasst ist, zu veranlassen, das der Prozessor (30) das Verfahren nach einem der Ansprüche 1 bis 8 durchführt.

15. Computerlesbares Medium, das das Computerprogrammelement nach Anspruch 14 darauf gespeichert aufweist.

## Revendications

1. Procédé mis en œuvre par ordinateur destiné à déterminer une métrique de santé d'un système d'imagerie (60), le procédé comprenant :
la réception (110) de données de capteur acquises au cours d'une séquence de diagnostic comprenant de multiples intervalles de diagnostic, dans lequel, pendant chacun des intervalles de diagnostic, un ensemble respectif de sous-systèmes du système d'imagerie (60) est actif et un sous-ensemble respectif de données de capteur est acquis, dans lequel les ensembles de sous-systèmes actifs pendant les intervalles de diagnostic respectifs sont mutuellement différents ; et
l'analyse (120) des sous-ensembles de données de capteurs pour déterminer la métrique de santé, dans lequel la détermination de la métrique de santé comprend l'exécution d'une opération mathématique entre au moins deux des sous-ensembles de données de capteur acquis pendant des intervalles de diagnostic mutuellement différents avec des ensembles mutuellement différents de sous-systèmes actifs.

2. Procédé selon la revendication 1, dans lequel un premier sous-ensemble et un second sous-ensemble comprennent des données de fréquence, et dans lequel l'opération mathématique comprend la soustraction de données de fréquence du premier sous-ensemble de données de fréquence du second sous-ensemble.

3. Procédé selon la revendication 1 ou 2, dans lequel le procédé comprend en outre la détermination et la production (130) d'un état de santé sur la base de la métrique de santé et/ou la détermination et la production (140) d'une prédiction de défaillance sur la base de la métrique de santé et/ou la détermination et la production (140) d'une recommandation sur la base de la métrique de santé.

4. Procédé selon la revendication 1, 2 ou 3, dans lequel le procédé comprend en outre l'analyse des données de capteur pour identifier automatiquement (112) le type et/ou la durée d'au moins un intervalle de diagnostic.

5. Procédé selon l'une quelconque des revendications précédentes, dans lequel le procédé comprend en outre la réception (114) d'un signal de déclenchement de système indicatif du type et/ou de la durée d'au moins un intervalle de diagnostic.

6. Procédé selon l'une quelconque des revendications précédentes, dans lequel des données de capteur comprennent des données provenant d'une mesure d'au moins un parmi
un son et/ou une vibration ;
une température ;
un courant de moteur d'anode de tube à rayons X ;
une émission de rayons X ; et
un mouvement de tube à rayons X.

7. Procédé selon l'une quelconque des revendications précédentes, dans lequel les intervalles de diagnostic comprennent au moins un parmi
un premier intervalle de silence ;
un intervalle de montée en puissance d'anode ;
un intervalle de montée en puissance de portique ou de bras en C ;
un intervalle de radiographie en cours ;
un intervalle de radiographie non prévue ;
un intervalle de décélération de portique ou de bras en C ;
un intervalle de décélération d'anode ; et
un second intervalle de silence.

8. Procédé selon l'une quelconque des revendications précédentes, dans lequel la métrique de santé comprend au moins un parmi
une modification de courant de moteur d'anode ;
une fréquence moyenne ;
des ordres entiers ;
des commandes non-entières ;
des tic-tac sonores ; et
une largeur de raie de rayons X mesurés.

9. Système de diagnostic (10) destiné à déterminer une métrique de santé d'un système d'imagerie (60), le système de diagnostic (10) comprenant :
un capteur (20) destiné à l'acquisition de données de capteur, et
un processeur (30) configuré pour mettre en œuvre le procédé selon l'une quelconque des revendications précédentes.

10. Système de diagnostic (10) selon la revendication 9, dans lequel le système de diagnostic (10) comprend une unité de communication (40) configurée pour communiquer au moins une parmi une métrique de santé, un état de santé, une prédiction de défaillance et une recommandation de service.

11. Système de diagnostic (10) selon la revendication 9 ou 10, dans lequel le capteur (20) est configuré pour être fixé à une source de rayons X.

12. Système de diagnostic (10) selon la revendication 9 ou 10 ou 11, dans lequel le capteur (20) comprend un ou plusieurs parmi un capteur piézoélectrique, un capteur MEMS, un capteur à ultrasons, un capteur de courant, un capteur à rayons X et un capteur gyroscopique.

13. Système d'imagerie (60) comprenant le système de diagnostic (10) selon l'une quelconque des revendications 9 à 12.

14. Élément de programme informatique qui, lorsqu'il est exécuté par un processeur (30), est adapté pour amener le processeur (30) à réaliser le procédé selon l'une quelconque des revendications 1 à 8.

15. Support lisible par ordinateur sur lequel est stocké l'élément de programme informatique selon la revendication 14.
